# EUROPÄISCHE PATENTSCHRIFT

(11) **EP 1 155 113 B1**
(45) Veröffentlichungstag und Bekanntmachung des Hinweises auf die Patenterteilung: **23.10.2002**
(21) Anmeldenummer: 99962161.8
(22) Anmeldetag: 25.11.1999
(51) Int. Cl.: C12M 3/00

(54) **VORRICHTUNG ZUM ZÜCHTEN VON MENSCHLICHEM ODER TIERISCHEM GEWEBE**
DEVICE FOR CULTURING HUMAN OR ANIMAL TISSUE
DISPOSITIF POUR LA CULTURE DE TISSU HUMAIN OU ANIMAL

(30) Priorität: 15.03.1999 DE 19911326
(43) Veröffentlichungstag der Anmeldung: 21.11.2001
(73) Patentinhaber: Fege, Wolfgang, Dr. med., 33014 Bad Driburg (DE)
(72) Erfinder: Fege, Wolfgang, Dr. med., 33014 Bad Driburg (DE)
(86) Internationale Anmeldenummer: EP9909126
(87) Internationale Veröffentlichungsnummer: WO00055299

(56) Entgegenhaltungen:
- WO-A-96/29865
- WO-A-96/40859
- DE-A- 3 712 200
- US-A- 5 376 548
- US-A- 5 773 285
- PATENT ABSTRACTS OF JAPAN vol. 011, no. 060 (C-405), 24. Februar 1987 (1987-02-24) & JP 61 218501 A (SENKO IKA KOGYO KK), 29. September 1986 (1986-09-29)

## Beschreibung

Die Erfindung betrifft eine Vorrichtung der im Oberbegriff des Anspruch 1 genannten Art.

Durch den Bericht "Tissue engineering: generation of differentiated artificial tissues for biomedical applications" von Minuth, Sittinger und Kloth, in Cell and tissue Research (1998) 291/1-11, Springer Verlag 1998, ist eine Vorrichtung der im Oberbegriff des Anspruchs 1 genannten Art bekannt. Bei dieser bekannten Vorrichtung werden Zellträger in die geöffnete Zellzüchtungskammer eingebracht und randseitig eingespannt. Nach Schließen der Kammer werden unterschiedliche Nährflüssigkeiten hinzugeführt, von denen eine oberhalb und die andere unterhalb des Zellträgers entlangströmt, auf dem so ein Gewebewachstum erfolgt, und zwar derart, daß verschiedene Gewebsausdifferenzierungen an der Unter- und Oberseite erzeugt werden, um zum Beispiel Haut zu züchten. Ein Nachteil der Zuchtkammer von Minuth besteht darin, daß das Gewebe nur die Form des Zellträgers (im konkreten Fall einem Plättchen) annimmt. Sollte es darüberhinauswachsen, würde spätestens, wenn eine derartige Züchtungskammer ab einer gewissen Größe ausgefüllt ist, was für eine durch die Kammerform bestimmte Formzüchtung unerläßlich ist, in Teilen des Gewebes zuwenig Interzellularsubstanz gebildet werden oder würden Teile sogar absterben, weil die Gewebeernährung mangels Perfusionsleistung und/oder Sauerstoffversorgung nicht mehr ausreichen würde. Ein weiterer Nachteil besteht in einer ungenügenden Formstabilität des zu züchtenden Gewebes bei mechanischer Belastung.

Aus der WO-A-96/40859 ist eine Vorrichtung zum Züchten von dreidimensionalem Gewebe, insbesondere Hautgewebe, bekannt. Die Vorrichtung besteht aus einem Behälter mit zwei miteinander verbindbaren Behälterteilen, welche eine Züchtungskammer bilden. Die Züchtungskammer weist eine oder mehrere untere Einlaßöffnungen zur Zufuhr und eine oder mehrere obere Auslaßöffnungen zur Ableitung von Mährflüssigkeit auf, die die Züchtungskammer vorzugsweise von unten nach oben durchströmt. Auf der Innenfläche der Behälterteile sind Gerüste oder Abstandselemente angeordnet, um einen Kontakt des Gewebes mit der Kammerwandung und auf diese Weise ein Zellwachstum auf der Kammerwandung zu verhindern. Es können innerhalb der Züchtungskammer Verteiler angeordnet sein, um die Nährflüssigkeit gleichmäßiger zu verteilen. Mit dieser bekannten Züchtungskammer kann das Gewebe nicht in eine bestimmte Form mit dreidimensionaler Oberflächenstruktur, sondern kann allenfalls zu einem ebenflächigen Gewebe, insbesondere Hautgewebe gezüchtet werden. Die Ernährung des Gewebes erfolgt über den Innenraum der Züchtungskammer. Eine gezielte Ernährung der Oberfläche des Gewebes ist nicht möglich.

Die DE-A-37 12 200 offenbart eine Vorrichtung zur extrakorporalen Langzeit-Perfusion von menschlichem oder tierischem Gewebe und zur extrakorporalen Regeneration von Organen. In dieser bekannten Vorrichtung befinden sich die Organe in einem speziellen Organträger, der aus einem Säckchen, das formschlüssig und gleichmäßig mit der gesamten Organoberfläche indirekt verbunden ist, und einem formfesten Behälter besteht, der druckdichte Durchführungen für die Anschlüsse der Organgefäße aufweist und einen Anschluß für das Druckmittel besitzt, das das Säckchen, welches im Behälter druckdicht angeordnet ist, entsprechend den physioligischen Druckverhältnissen des Organs im Körper beaufschlagt. Die bekannte Vorrichtung ist nicht zum Züchten von Gewebe vorgesehen und geeignet. Eine gezielte Nährflüssigkeitsszufuhr und/oder -abfuhr in/aus verschiedenen Bereichen der Kammer ist nicht möglich.

Die Aufgabe der vorliegenden Erfindung besteht darin, eine Vorrichtung der betreffenden Art zu schaffen, mit der Gewebe in einer gewünschten und stabilen Form und/oder Oberflächenausbildung gezüchtet werden kann.

Die der Erfindung zugrundeliegende Aufgabe wird durch die im Kennzeichen des Anspruchs 1 angegebene Lehre gelöst.

Zur Lösung der Aufgabe schlägt die Erfindung somit eine Züchtungskammer vor, deren wenigstens eine Innenfläche eine dreidimensionale Form aufweist und die eine Negativform einer zu züchtenden Gewebeform bzw. eines zu züchtenden Körperteiles ist. Mit der erfindungsgemäßen Züchtungskammer kann somit ein Gewebe in eine bestimmte dreidimensionale Form insbesondere mit dreidimensionaler Oberflächenstruktur gezüchtet werden, wobei die Zufuhr von Nährflüssigkeit zum Gewebe über die das Gewebe formende Innenfläche der Züchtungskammer zugeführt wird, in die Kanäle zur Zufuhr und Ableitung der Nährflüssigkeit münden, wobei eine gezielte Nährflüssigkeitszufuhr und/oder -abfuhr in/aus verschiedenen Bereichen der Züchtungkammer ermöglicht wird. Die Zahl und die Lage der einzelnen Kanäle kann beliebig variiert werden, um so das Gewebewachstum in den jeweils erfaßten Bereichen zu beeinflussen. Das zu züchtende Gewebe oder der zu züchtende Körperteil erhält beim Züchtungsvorgang unmittelbar die gewünschte Form, bspw. eines Ohr- oder Nasenknorpels, welcher dann unmittelbar implantiert werden kann. Es können auch vorgezüchtete Geweberohlinge in der Züchtungskammer in eine Form gepreßt und in diese Form gezüchtet werden. Dies kann auch unter Zugabe von synthetischen oder anderen extrakorporal vorgezüchteten Gewebeformteilen geschehen.

Zweckmäßigerweise sind die Behälterteile von einem Gehäuse dicht umschlossen, das die Züchtungskammer vollständig einschließt und durch das mit den Kanälen verbundene Zu- und/oder Abführungsleitungen für die Nährflüssigkeit einzeln oder in einem oder mehreren Bündeln zusammengefaßt hindurchgeführt sind. Auf diese Weise ist durch das Gehäuse ein zusätzlicher Schutz vor Keimbesiedelung erreichbar.

Wenn gemäß einer Weiterbildung der Erfindung in den Verbindungsleitungen Mengenregler angeordnet sind, ist es zusätzlich möglich, Geschwindigkeit und Menge der den einzelnen Bereichen zugeführten Nährflüssigkeit anzupassen.

Bei großer Dichte der Kanäle kann deren Herstellung schwierig sein. Aus diesem Grund sieht eine zweckmäßige Ausführungsform gemäß der Erfindung vor, daß die Kanäle durch offene Poren eines offenporigen Schaumstoffs oder eines Gels gebildet sind.

Um das gezüchtete Gewebe sicher innerhalb der Züchtungskammer zu halten, sieht eine Weiterbildung der Erfindung vor, auf der Innenfläche wenigstens eines der Behälterteile ein Filterelement anzuordnen, das eine oder mehrere Filterschichten aufweist.

Sind die Innenflächen der Behälterteile dreidimensional, so ist es zweckmäßig, auch in diesem Fall die Innenflächen der Behälterteile durch jeweils in gleicher Weise dreidimensional geformte Filterelemente abzudecken, die ebenfalls eine oder mehrere Filterschichten aufweisen können.

Mit der erfindungsgemäßen Vorrichtung können grundsätzlich Gewebeteile mit oder ohne Stützgerüst und mit oder ohne Zellträger in eine Form gezüchtet werden. Das Stützgerüst und/oder der Zellträger wird dann einfach bei Beginn des Züchtungsvorganges, bereits mit Zellen bedeckt und/oder hiermit durchsetzt, in die Züchtungskammer gebracht. Ist die Züchtungskammer dabei gemäß einer erfindungsgemäßen Ausführungsform entsprechend einem zu züchtenden Körperteil dreidimensional geformt, so ergeben sich zwangsläufig auf dem Stützgerüst unterschiedlich dicke Gewebequerschnitte. Dies kann dazu führen, daß die Ernährung des Gewebes teilweise unzureichend ist, mit der Folge, daß das Gewebe abstirbt, oder aber die Festigkeit der Gewebestruktur an einzelnen Stellen und/oder insgesamt leidet.

Aus diesem Grund sieht die Erfindung vor, daß die Züchtungskammer von einem Perfusionsröhrchen oder mehreren Perfusionsröhrchen durchsetzt ist/sind zur Zu- und/oder Abfuhr von Nährflüssigkeit, wobei das Röhrchen bzw. die Röhrchen durch das Zuchtgewebe hindurchführt/hindurchführen. Vorzugsweise bestehen die Röhrchen aus zwei steckbaren und damit trennbaren Teilen oder bestehen wenigstens an einer Stelle aus auflösbarem Material, sodaß sie nach Abschluß der Züchtung zum Erhalt der Sterilität des Gewebeblockes zu beiden Seiten herausgezogen werden können.

Wenn der innerhalb des Gewebeblockes befindliche Teil des Röhrchens so beschaffen ist, daß er sich auflöst, muß das Röhrchen nicht zweigeteilt sein; es werden dann vor Implantation lediglich die äußeren Enden abgetrennt oder aus dem Gewebeblock herausgezogen.

Gemäß einer Weiterbildung der Erfindung ist/sind das Perfusionsrohr/die Perfusionsröhrchen auf seiner Außenfläche/ihrer Außenflächen wenigstens im Bereich der Durchbrüche durch wenigstens ein Filterelement, das eine oder mehrere Filterschicht/-en aufweisen kann, abgedeckt.

Mit der erfindungsgemäßen Vorrichtung ist es möglich, dem zu züchtenden Gewebe von außen eine Form zu verleihen, die es auch unter späterer mechanischer Belastung, zum Beispiel nach Implantation, beibehält. Bei dieser Formung von außen wird auch die gewünschte Form erzielt und/oder während der Gewebezüchtung beibehalten, wenn mechanische Einflüsse von außen, wie zum Beispiel Druckschwankungen (Ultraschall) oder Rotationen einwirken.

Gemäß einer weiteren Ausgestaltung der Erfindung weist die dem zu züchtenden Gewebe zugewandte Oberfläche des Filterelementes eine Höhenstruktur auf, welche dem zu züchtenden Gewebe eine entsprechend komplementäre Oberflächenausbildung verleiht, was für das Einwachsen von großer Bedeutung ist. So kann zum Beispiel ein schnelles und/oder festes Einwachsen von bestimmten Teilen und ein verzögertes und/oder lockeres Einwachsen von anderen Teilen vorausbestimmt werden.

Gemäß einer weiteren Ausgestaltung der Erfindung besitzt das Filterelement auf der dem Zuchtgewebe abgewandten Seite eine weitere oder mehrere weitere Filterschichten, die in tangentialer Richtung offenporig ausgebildet ist/sind und/oder Kanäle aufweist/aufweisen. Durch diese Ausbildung kann die Verteilung der Nährlösung verbessert werden.

Anhand der Zeichnung soll die Erfindung an Ausführungsbeispielen näher erläutert werden.
- Fig. 1: zeigt im Schnitt ein erstes Ausführungsbeispiel, und
- Fig. 2: zeigt schematisch ein zweites Ausführungsbeispiel der Erfindung.

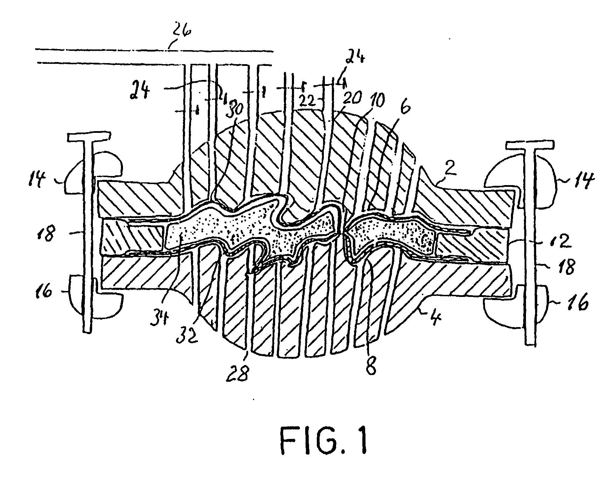

Fig. 1 zeigt im Schnitt zwei Behälterteile 2 und 4, die zwischen ihren dreidimensional geformten Innenflächen 6 und 8 eine dreidimensionale Züchtungskammer 10 einschließen.

Zwischen den Rändern der Behälterteile 2 und 4 befindet sich eine Dichtung 12, gegen die die Ränder der Behälterteile 2 und 4 durch Klemmbacken 14 und 16 gepreßt sind, so daß die Züchtungskammer 10 seitlich dicht verschlossen ist. Die Klemmbacken 14 und 16 sind durch eine schematisch dargestellte Spannschraube 18 festziehbar.

In beiden Behälterteilen 2 und 4 befinden sich Kanäle 20, 28, die jeweils durch Verbindungsleitungen 22 und als Ventile ausgebildete Mengenregler 24 mit einer gemeinsamen Leitung 26 oder mit unterschiedlichen Leitungen zur Zuführung und/oder Abführung von Nährflüssigkeit verbunden sind. Aus Gründen der Übersichtlichkeit sind nur einige der Verbindungsleitungen 22 und Mengenregler 24 gezeigt; natürlich sind alle Kanäle 18, 20 in gleicher Weise an die gemeinsame Leitung oder verschiedene Leitungen 26 angeschlossen.

Die Innenflächen 6 und 8 der Behälterteile 2 und 4 sind über ihre gesamte Ausdehnung durch komplementär zu den Innenflächen 6 und 8 dreidimensional geformte Filterelemente 30 und 32 abgedeckt, die sich in den Bereich der Dichtung 12 erstrecken, so daß in der Züchtungskammer 10 befindliches, schematisch dargestelltes Zuchtgewebe 34 vollständig von den Filterelementen 30, 32 umschlossen und somit von den Zu- und Abflüssen der Kanäle 20, 28 getrennt ist. Die Filterelemente 30, 32 haben außerdem die Aufgabe, die durch die Kanäle 20 und/oder 28 zuströmende Nährflüssigkeit gleichmäßig der Oberfläche des Zuchtgewebes 34 zuzuführen. Dies gilt in entsprechender Weise für den Abfluß in die Kanäle 20 und/oder 28.

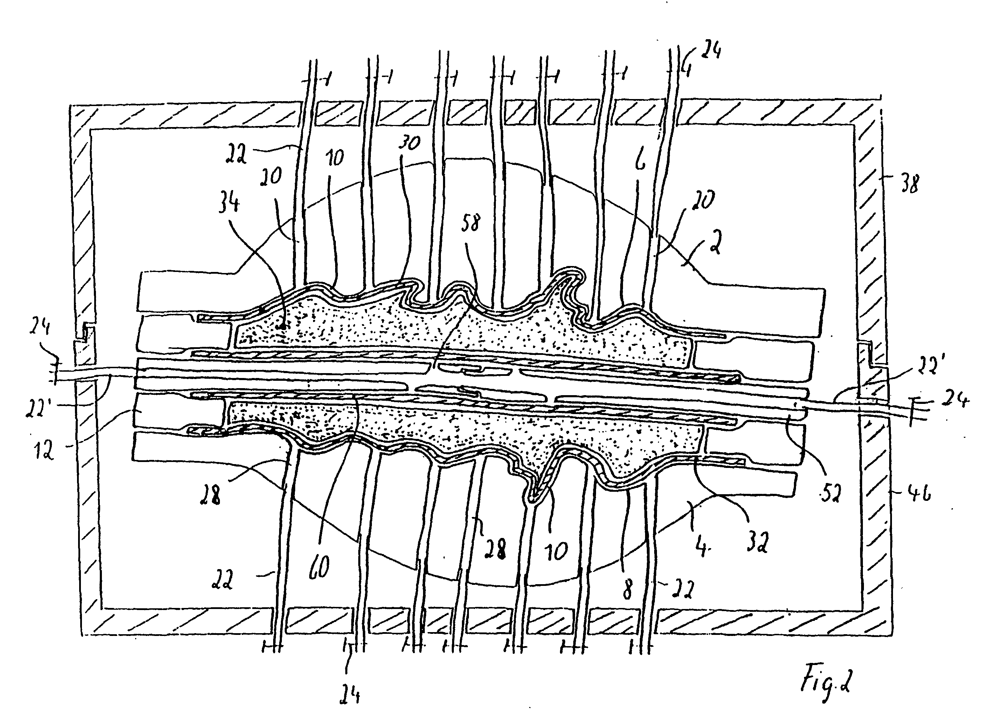

Fig. 2 zeigt ein Ausführungsbeispiel ähnlich gemäß Fig. 1. Gleiche oder sich entsprechende Teile sind mit gleichen Bezugsziffern versehen, so daß insofern auf die Beschreibung der Fig. 1 verwiesen wird.

Die Kanäle 20, 28 zur Zu- und/oder Abführung der Nährflüssigkeit führen durch die Wandung der Behälterteile 2 und 4 hindurch. Die Behälterteile 2, 4 sind von einem Gehäuse 38, 46 umschlossen, das die Züchtungskammer 10 voll einschließt. Durch das Gehäuse 38, 40 sind mit den Kanälen verbundene Zu- und/oder Abführungsleitungen 22 für die Nährflüssigkeit einzeln oder in einem oder in mehreren Bündeln zusammengefaßt hindurchgeführt.

Die Züchtungskammer 10 ist von einem Perfusionsrohr 52 oder von mehreren Perfusionsrohren durchsetzt, das bzw. die durch das zu züchtende Gewebe 34 hindurchgeführt ist/sind. Das Perfusionsrohr 52 dient zur Versorgung des Zuchtgewebes mit Nährflüssigkeit von innen. Hierzu weist das Perfusionsröhrchen 52 vorzugsweise Öffnungen 58 auf, über die es in offener Verbindung mit der Zuchtkammer 10 steht. Durch das Gehäuse 38, 40 sind mit dem Perfusionsrohr 52 verbundene Zu- und/oder Abführungsleitungen 22' hindurchgeführt.

Wie in der Figur 2 dargestellt, besteht das Perfusionsröhrchen 52 vorzugsweise aus zwei steckbaren und damit trennbaren Teilen, sodaß diese nach Abschluß der Züchtung zum Erhalt der Sterilität des Gewebeblockes zu beiden Seiten herausgezogen werden können. Das Perfusionsröhrchen kann aber auch so ausgebildet sein, daß es wenigstens in einem Bereich auflösbar ist, sodaß es ebenfalls beidendig nach Abschluß der Züchtung herausgezogen werden kann.

Das Perfusionsröhrchen 52 kann auf seiner Außenfläche wenigstens im Bereich der Öffnungen 58 durch ein Filterelement 60 abgedeckt sein, das ein oder mehrere Filterschichten aufweist.

Das Filterelement 60 kann auf der dem Zuchtgewebe abgewandten Seite eine weitere oder mehrere weitere Filterschichten aufweisen, die in tangentialer Richtung offenporig ausgebildet ist/sind und/oder Kanäle aufweist/aufweisen zur Erzielung eines Nährlösungsstromes tangential zur Gewebeoberfläche, wodurch eine bessere Verteilung der Nährlösung erreicht werden kann. Eine derartige Ausbildung können auch die Filterelemente 30, 32 auweisen.

Die dem zu züchtenden Gewebe zugewandte Oberfläche des Filterelementes 60 und/oder der Filterelemente 30, 32 kann eine bestimmte Höhenstruktur aufweisen (nicht dargestellt), welche dem zu züchtenden Gewebe eine entsprechend komplementäre Oberflächenausbildung verleiht. Die Höhenstruktur kann örtlich unterschiedlich sein. Dies kann für das Einwachsen von großer Bedeutung sein. So kann zum Beispiel ein schnelles und/oder festes Einwachsen von bestimmten Teilen und ein verzögertes und/oder lockeres Einwachsen von anderen Teilen vorausbestimmt werden.

## Patentansprüche

1. Vorrichtung zum Züchten von menschlichem oder tierischem Gewebe, mit wenigstens zwei Behälterteilen, die dicht miteinander verbindbar sind und zwischen denen sich eine Züchtungskammer befindet, und mit Zuführmitteln zur Zuführung von Nährflüssigkeit und mit Ableitungsmitteln zur Ableitung der Nährflüssigkeit oder umgekehrt, **dadurch gekennzeichnet, daß** die Zuführungs- und Abführungsmittel wenigstens einen Kanal oder mehrere Kanäle aufweisen, welcher/welche als ein oder mehrere Perfusionsröhrchen (**52**) die Züchtungskammer (**10**) durchsetzen und mit dem/denen Zu- und/oder Abführungsleitungen (**22'**) verbunden sind, zur gezielten Zu- und/oder Abfuhr von Nährflüssigkeit von innen und einen oder mehrere Kanal/Kanäle (**20,28**) aufweisen, der/die durch die Wandung eines Behälterteils (**2,4**) hindurchführt/hindurchführen und in einer Innenfläche (**6,8**) eines Behälterteils (**2,4**) mündet/münden, so daß eine gezielte Nährflüssigkeitszufuhr und/oder -abfuhr ermöglicht wird, um so das Gewebewachstum in den jeweils erfassten Bereichen zu beeinflussen.

2. Vorrichtung nach Anspruch 1, **dadurch gekennzeichnet, daß** die Kanäle (20, 28) mit im wesentlichen gleichem Abstand oder unterschiedlichem Abstand zueinander angeordnet sind.

3. Vorrichtung nach Anspruch 1, **dadurch gekennzeichnet, daß** die Kanäle durch offene Poren eines offenporigen Schaumstoffs oder eines Gels gebildet sind.

4. Vorrichtung nach Anspruch 1, **dadurch gekennzeichnet, daß** auf der Innenfläche (6, 8) wenigstens eines der Behälterteile (2, 4) ein Filterelement (30, 32), das eine Filterschicht oder mehrere Filterschichten aufweist, angeordnet ist.

5. Vorrichtung nach Anspruch 1, **dadurch gekennzeichnet, daß** die Innenflächen (6, 8) der Behalterteile (2, 4) durch jeweils in gleicher Weise dreidimensional geformte Filterelemente (30, 32), die eine Filterschicht oder mehrere Filterschichten aufweisen, abgedeckt sind.

6. Vorrichtung nach Anspruch 1, **dadurch gekennzeichnet, daß** der Innenraum des Perfusionsröhrchens (52) oder der Perfusionsröhrchen über im Mantel der Röhrchen ausgebildete Öffnungen (58) in offener Verbindung mit der Züchtungskammer (10) steht.

7. Vorrichtung nach Anspruch 1 oder 6, **dadurch gekennzeichnet, daß** das Perfusionsröhrchen (52) oder die Perfusionsröhrchen in zwei oder mehreren Teilen trennbar ausgebildet ist/sind.

8. Vorrichtung nach Anspruch 1 oder 6, **dadurch gekennzeichnet, daß** das Perfusionsröhrchen (52) oder die Perfusionsröhrchen wenigstens teilweise aus einem sich auflösenden Material bestehen.

9. Vorrichtung nach einem der Ansprüche 1 oder 6 bis 8, **dadurch gekennzeichnet, daß** das Perfusionsröhrchen (52) oder die Perfusionsröhrchen auf seiner/ihrer AuBenfläche wenigstens im Bereich der Öffnungen (58) durch wenigstens ein Filterelement (60), das eine oder mehrere Filterschichten aufweist, abgedeckt ist/sind.

10. Vorrichtung nach Anspruch 4, 5 oder 9, **dadurch gekennzeichnet, daß** die dem zu züchtenden Gewebe zugewandte Oberfläche des Filterelementes (60) und/oder der Filterelemente (30, 32) eine Höhenstruktur aufweist, welche dem zu züchtenden Gewebe eine entsprechend komplementäre Oberflächenausbildung verleiht.

11. Vorrichtung nach Anspruch 10, **dadurch gekennzeichnet, daß** die Höhenstruktur örtlich unterschiedlich ist.

12. Vorrichtung nach Anspruch 10, **dadurch gekennzeichnet, daß** das Filterelement (60) und/oder die Filterelemente (30, 32) auf der dem Zuchtgewebe abgewandten Seite eine weitere Filterschicht oder mehrere weitere Filterschichten besitzt/besitzen, die in tangentialer Richtung offenporig ausgebildet ist/sind und/oder Kanäle aufweist/aufweisen.

## Claims

1. Device for cultivation of human or animal tissue, with at least two containerparts, which are connectable tightly and between them a cultivation chamber is situated, and with supply means for supplying with nutrient solution and with drawing off means for drawing off the nutrient solution or reversed, marked thereby, that the supply and drawing off means show at least one canal or several canals, which intersperse the cultivation chamber (**10**) as one or several perfusiontubes (**52**) and with that/them are connected supply and/or drawing off conductings (**22'**), for the well-aimed supply and/or drawing off of nutrient solution from inside and show one or several canal/canals (**20,28**), which leads/lead through the side of a container part (**2,4**) and ends/end in a interior surface (**6,8**) of a container part (**2,4**), so that a well-aimed supply of nutrient solution and/or drawing off is made been possible, to influence the tissue growth in each captured area in this way.

2. Device of claim 1. **marked thereby,** that the canals (20, 28) are arranged basically at an equal distance or at a different distance to each other.

3. Device of claim 1. **marked thereby,** that the canals are formed by open pores of an openporous foam material or a gel.

4. Device of claim 1. **marked thereby,** that on the inner surface (6, 8) of at least one of the container parts (2, 4) a filter element (30, 32) is arranged, which shows one filter layer or several filter layers.

5. Device of claim 1. **marked thereby,** that the inner surfaces (6, 8) of the container parts (2, 4) are at any one time covered by in the same way threedimensional formed filter elements (30, 32), which show one filter layer or several filter layers.

6. Device of claim 1. **marked thereby,** that the inner rooms of the perfusion tube (52) or the perfusion tubes are open connected with the cultivation chamber (10) by openings (58) formed in the casing of the tubes.

7. Device of claim 1. **marked thereby,** that the perfusion tube (52) or the perfusion tubes is/are formed separable in two or several parts.

8. Device of claim 1 or 6 **marked thereby,** that the perfusion tube (52) or the perfusion tubes consist of an at least partly dissolving material.

9. Device of one of the claims 1 or 6 to 8, **marked thereby,** that the perfusion tube (52) or the perfusion tubes are covered on its/their outer surface at least in the area of the openings (58) by at least one filter element (60), which shows one or several filter layers.

10. Device of claim 4, 5 or 9 **marked thereby,** that the surface of the filter element (60) and/or of the filter elements (30, 32) facing the tissue to be cultivated show a structure of the altitude level, which lends to the tissue to be cultivated a corresponding complementary formation of the surface.

11. Device of claim 10 **marked thereby,** that the structure of the altitude level is locally different.

12. Device of claim 10 **marked thereby,** that the filter element (60) and/or the filter elements (30, 32) one the side averted from the cultivation-tissue possess one further filter layer or several further filter layers, which is/are formed openporous in tangential direction and/or shows/show canals.

## Revendications

1. Dispositif destiné à la culture de tissus humain ou animaux, avec au moins deux parties de récipient pouvant être reliées entre elles de manière étanche et entre lesquelles se situe une chambre de culture, et avec des moyens d'alimentation pour l'alimentation en liquide nutritif et des moyens d'évacuation pour l'évacuation du liquide nutritif ou vice versa, **caractérisée en ce que** les moyens d'alimentation et d'évacuation présentent au moins un canal ou plusieurs canaux qui entrepassent la chambre de culture (**10**) comme un ou plusieurs tubes capillaires de perfusion (**52**) et sont reliés avec les conduites d'alimentation et d'évacuation (**22'**) pour permettre l'alimentation ou l'évacuation ciblée du liquide nutritif de l'intérieur et qui présentent un canal ou plusieurs canaux **(20, 28)** traversant la paroi d'une partie du récipient (**2, 4**) et aboutissant dans une des surfaces internes (**6, 8**) d'une partie du récipient (**2, 4)**), de façon à permettre une alimentation ciblée en liquide nutritif et/ou l'évacuation de celui-ci, afin d'influencer ainsi la croissance du tissu dans les secteurs respectivement desservis.

2. Dispositif selon la revendication 1, **caractérisé en ce que** les canaux (20, 28) sont disposés pour l'essentiel à intervalle régulier les uns des autres ou bien à intervalles différents.

3. Dispositif selon la revendication 1, **caractérisé en ce que** les canaux sont formés par les pores ouverts d'une mousse alvéolaire à pores ouverts ou par ceux d'un gel.

4. Dispositif selon la revendication 1, **caractérisé en ce que** sur la surface interne (6, 8) d'au moins une des parties du récipient (2, 4) est disposé un élément filtrant (30, 32) qui présente une couche filtrante ou plusieurs couches filtrantes.

5. Dispositif selon la revendication 1, **caractérisé en ce que** les surfaces internes (6, 8) des parties de récipient (2, 4) sont chacune recouvertes d'éléments filtrants (30, 32) formés de la même façon de manière tridimensionnelle et présentant une couche filtrante ou plusieurs couches filtrantes.

6. Dispositif selon la revendication 1, **caractérisé en ce que** l'intérieur du tube capillaire de perfusion (52) ou des tubes capillaires de perfusion est en liaison ouverte avec la chambre de culture (10) par l'intermédiaire d'orifices (58) situés dans la gaine du tube capillaire.

7. Dispositif selon les revendications 1 ou 6, **caractérisé en ce que** le tube capillaire de perfusion (52) ou les tubes capillaires de perfusion est/sont formé(s) de deux ou plusieurs parties séparables.

8. Dispositif selon les revendications 1 ou 6, **caractérisé en ce que** le tube capillaire de perfusion (52) ou les tubes capillaires de perfusion sont constitués au moins en partie de matière soluble.

9. Dispositif selon les revendications 1 ou 6 à 8, **caractérisé en ce que** la surface extérieure du tube capillaire de perfusion (52) ou des tubes capillaires de perfusion est recouverte au moins au niveau des orifices (58) par au moins un élément filtrant (60) présentant une ou plusieurs couches filtrantes.

10. Dispositif selon les revendications 4, 5 ou 9, **caractérisé en ce que** la surface de l'élément filtrant (60) tournée vers le tissu à cultiver et/ou celle des éléments filtrants (30, 32) présente une structure en hauteur conférant au tissu à cultiver une forme correspondante de surface complémentaire.

11. Dispositif selon la revendication 10, **caractérisé en ce que** la structure en hauteur présente des différences locales.

12. Dispositif selon la revendication 10, **caractérisé en ce que** l'élément filtrant (60) et/ou les éléments filtrants (30, 32) situés possède/possèdent, du côté opposé au tissu à cultiver, une autre couche filtrante ou plusieurs autres couches filtrantes qui est/sont formée(s) de manière tangentielle à pores ouverts et présente/présentent des canaux.
